(19) Europäisches Patentamt
European Patent Office
Office européen des brevets

(11) EP 0 761 210 A2

(12) **EUROPÄISCHE PATENTANMELDUNG**

(43) Veröffentlichungstag:
12.03.1997 Patentblatt 1997/11

(51) Int. Cl.⁶: **A61K 9/22**

(21) Anmeldenummer: 96113158.8

(22) Anmeldetag: 16.08.1996

(84) Benannte Vertragsstaaten:
AT BE CH DE DK ES FI FR GB GR IE IT LI LU MC NL PT SE

(30) Priorität: 29.08.1995 DE 19531684

(71) Anmelder: **BAYER AG**
51368 Leverkusen (DE)

(72) Erfinder:
- **Kanikanti, Venkata-Rangarao, Dr.**
  51381 Leverkusen (DE)
- **Kettelhoit, Stefan, Dr.**
  51375 Leverkusen (DE)
- **Kurka, Peter, Dr.**
  40764 Langenfeld (DE)
- **Penners, Gunther, Dr.**
  51373 Leverkusen (DE)
- **Serno, Peter, Dr.**
  51467 Bergisch Gladbach (DE)

(54) **Verfahren zur Herstellung von Arzneimittelzubereitungen mit kontrollierter Freisetzung**

(57) Die Erfindung betrifft ein spezielles Verfahren zur Herstellung von stabilen, festen Arzneizubereitungen mit hoher Bioverfügbarkeit und kontrollierter Freisetzung enthaltend schwerlösliche Wirkstoffe und polymere Hilfsstoffe und die so hergestellten Zubereitungen.

Printed by Rank Xerox (UK) Business Services
2.13.16/3.4

**Beschreibung**

Die Erfindung betrifft ein spezielles Verfahren zur Herstellung von stabilen, festen Arzneizubereitungen mit hoher Bioverfügbarkeit und kontrollierter Freisetzung enthaltend schwerlösliche Wirkstoffe und polymere Hilfsstoffe und die so hergestellten Zubereitungen.

Feste Lösungen oder Copräzipitate aus Wirkstoffen wie z.B. Dihydropyridinen und Polyvinyllactamen sind bekannt. Die Herstellung kann z.B. durch gemeinsames Lösen des Wirkstoffes und Hilfsstoffes in organischen Lösungsmitteln und Weiterverarbeitung zu Tabletten erfolgen oder durch Formgebung einer Wirkstoff-Hilfsstoffschmelze, z.B. durch Spritzguß oder Extrusion und nachfolgender Kalandrierung.

Die Herstellung über organische Lösungen beinhaltet folgende Probleme: Geeignete organische Lösungsmittel wie chlorierte Kohlenwasserstoffe, Ketone oder Alkohole stellen eine Umweltbelastung dar und müssen daher während oder nach der Herstellung fester Lösungen mit großem technischen Aufwand zurückgewonnen werden. Bei der Verwendung von Lösungsmitteln, die mit Luft explosions- oder zündfähige Gemische bilden, muß der gesamte Prozeß unter Explosionsschutz verlaufen. Es werden hohe Hilfsstoffmengen benötigt, da die organische Lösung von Wirkstoff und Polymer zur weiteren Arzneiformung auf einen Träger, beispielsweise einem quer-vernetzten Polymer, aufgezogen werden muß (vgl. EP 0 232 155). Die vollständige Entfernung des verwendeten Lösungsmittels aus dem Produkt ist zumeist aufwendig oder unmöglich, so daß ein unerwünschter Gehalt an Restlösungsmitteln im Produkt verbleibt. So hergestellte Copräzipitate neigen häufig zur Rekristallisation des Wirkstoffs und sind daher nicht lagerstabil bzw. zeigen keine gleichbleibende Bioverfügbarkeit über mehrere Monate Lagerzeit.

Die Herstellung fester Lösungen durch innige Vermischung des Wirkstoffes mit einer Polymerschmelze und dem nachfolgenden Verarbeiten der Schmelze unter Formgebung zu festen Teilen (z.B. EP 0 596 203) hat sich ebenfalls als nachteilig herausgestellt.

Erfolgt die Vermischung des Wirkstoffes mit der Schmelze in Ein- und Doppelschneckenextrudern und die nachfolgende Formgebung des Extrudates auf Kalanderwalzen, so bestehen verfahrenstechnische Limitierungen der erzielbaren Form der Teilchen. Insbesondere ist die dem Patienten bekannte zylindrische Tablettenform mit seitlichem Steg nicht oder nur sehr eingeschränkt herstellbar. Dies hat eine verringerte Akzeptanz der Medikation zur Folge und birgt die Gefahr, daß die erhaltenen Teilchen mit rundem oder kissenförmigem Querschnitt mit Süßigkeiten verwechselt werden.

Erfolgt die Vermischung des Wirkstoffes mit der Schmelze und Formgebung im Spritzgußverfahren, so müssen andere Nachteile wie beispielsweise eine geringe Taktzahl des Verfahrens hingenommen werden.

Darüber hinaus hat das in EP 0 596 203 beschriebene Verfahren der innigen Vermischung eines Wirkstoffes mit einer Polymerschmelze und nachfolgender Formgebung erhebliche Limitierungen bezüglich der verwendbaren Polymere. So müssen - wie dort beschrieben - die eingesetzten Polymere innerhalb bestimmter Viskositätsstufen liegen. Auch muß zumindest eines der beiden eingesetzten Polymere eine ausreichend niedrige Viskosität in geschmolzener Form aufweisen, so daß eine Formgebung überhaupt noch möglich ist. Als Folge dieser Limitierungen können nicht die Polymere eingesetzt werden, die im Sinne einer effektiven Freisetzungssteuerung optimal wären und es resultieren vergleichweise große und somit für den Patienten nachteilige Arzneiformen.

Der vorliegenden Erfindung lag die Aufgabe zugrunde, die im Vorangehenden genannten verfahrenstechnischen Einschränkungen zu überwinden. Die Probleme werden gelöst durch das neue Verfahren, das die Herstellung von Arzneizubereitungen durch Granulation oder Tablettierung von Pulvern oder Granulaten nach üblichen Methoden nachfolgend eine thermische Auflösung oder Amorphisierung des Wirkstoffs betrifft. Dabei erlaubt die Formgebung des ersten Verfahrensschrittes durch Verpreßen (Tablettieren) oder Granulieren eine erheblich vorteilhaftere Auswahl der Polymere. Die fertiggeformte Arzneizubereitung wird dann in einem folgenden, einfach durchzuführenden Prozeßschritt erhitzt.

Die Erfindung betrifft somit die Herstellung von festen Arzneizubereitungen enthaltend 1 Gew.-Teil eines oder mehrerer schwer löslicher Wirkstoffe (I), 0,5 bis 50 Gew.-Teile, vorzugsweise bis 20 Gew.-Teile, eines Celluloseethers (II) und 0,5 bis 50 Gew.-Teile, vorzugsweise bis 20 Gew.-Teile, Polyvinyllactame oder Polyvinyllactam-Copolymere (III) sowie gegebenenfalls weitere übliche Hilfsstoffe, dadurch gekennzeichnet, daß man die Bestandteile (I), (II) und (III) in Abwesenheit von Lösungsmitteln mischt und zu den gewünschten Arzneizubereitungen formt und diese anschließend über mindestens 30 Minuten bei Temperaturen zwischen 50 bis 200°C, vorzugsweise bei 80 bis 170°C, thermisch aktiviert.

Als bevorzugte feste Arzneizubereitungen seien Tabletten, Pellets und Granulate, insbesondere Tabletten genannt.

Die Dauer der thermischen Behandlung beträgt mindestens 30 Minuten und kann je nach gewählter Temperatur bis zu 3 Tagen, vorzugsweise bis zu 48 Stunden betragen.

Die erfindungsgemäß eingesetzten Celluloseether (II) sind bevorzugt Verbindungen auf Basis von linearen alkoxylierten Celluloseverbindungen die in Mengen zwischen 5 und 60 Gew.-%, vorzugsweise zwischen 20 und 45 Gew.-% bezogen auf das Gewicht der verbrauchsfertigen Zubereitung, eingesetzt werden. Besonders bevorzugte Celluloseether sind Verbindungen mit Methoxy- und Hydroxypropylsubstituenten die in 2 %igen wäßrigen Lösungen eine Viskosität von 15 bis 100 000 mPa·s, vorzugsweise 50 bis 30 000 mPa·s besitzen, beispielsweise die unter den

2

Handelsnamen bekannten Produkte METOLOSE 60 SH, METOLOSE 65 SH und METOLOSE 90 SH.

Die erfindungsgemäß eingesetzten Polyvinyllactame (III) sind vorzugsweise hochmolekulare Verbindungen auf Basis von Polyvinylpyrrolidon oder Vinylpyrrolidon-Vinylacetat-Copolymerisat die ein mittleres Molgewicht (Gewichtsmittelwert $\overline{M}$w) von 20 000 bis 2 000 000, vorzugsweise 25 000 bis 1 500 000 $\overline{M}$w besitzen und die in Mengen zwischen 5 und 60 Gew.-%, vorzugsweise zwischen 20 und 40 Gew.-%, bezogen auf das Endgewicht der Arzneizubereitung eingesetzt werden. Besonders bevorzugt sind lineare Polyvinylpyrrolidone mit einem hohen Molekulargewicht beispielsweise die unter den Handelsnamen KOLLIDON K25, KOLLIDON 90 und KOLLIDON VA 64 bekannt sind.

In die erfindungsgemäß hergestellten Zubereitungen können alle thermisch stabilen Wirkstoffe eingearbeitet werden. Vorzugsweise seien schwerlösliche Wirkstoffe, insbesondere solche aus der Gruppe der Dihydropyridine genannt. Anteilsmäßig werden diese Wirkstoffe vorzugsweise in Mengen von 5 bis 50 Gew.-%, vorzugsweise von 20 bis 35 Gew.-% der fertigen Zubereitung eingesetzt.

Im Gegensatz zu den bisher bekannten Verfahren zur lösungsmittelfreien thermischen Herstellung von festen Lösungen, bei denen immer die Notwendigkeit einer intensiven mechanischen Durchmischung des Wirkstoffs mit der Polymerschmelze, z.B. durch Schneckenextruder, bestand, wird in der vorliegenden Erfindung erstmals der Weg beschritten eine feste Lösung aus einem vorgeformten System eines Pulverkomprimates oder Granulates ohne weitere mechanische Einwirkung durch thermische Behandlung herzustellen. Dieses Verfahren hat den Vorteil, daß relativ einfache und bisher übliche Herstellungsgeräte wie Tablettiermaschinen oder Trockengeräte verwendet werden können. Ferner erlaubt das Verfahren die Auswahl möglichst effektiver Polymere ohne Rücksicht auf die Notwendigkeit zur Formgebung der Schmelze. Als Folge können wesentlich kleinere und leichtere Tabletten hergestellt werden, mit den Vorteilen der leichteren Schluckbarkeit für den Patienten, der Einsparung pharmakologische inaktiver Hilfsstoffe und auch kleiner Tablettenpackungen, die weniger Lagerfläche und Transportraum beanspruchen. Darüber hinaus weisen die erfindungsgemäß hergestellten Zubereitungen aufgrund des Fehlens von unerwünschten Lösungsmittelresten und geringen Hilfsstoffmengen auch pharmakologische Vorteile auf.

Von den mit Lösungsmitteln hergestellten Copräzipitaten ist bekannt, daß diese bei längerer Lagerung insbesondere unter ungünstigen Bedingungen wie z.B. hoher Luftfeuchtigkeit oder erhöhter Temperatur (Tropen) bereits nach kurzer Zeit eine Rekristallisation der schwerlöslichen Wirkstoffe zeigen, was zu einer Verringerung der Bioverfügbarkeit führt. Die erfindungsgemäß thermisch aktivierten lösungsmittelfreien Zubereitungen zeigen eine deutlich höhere Lagerstabilität und eine über mehrere Monate gleichbleibende gute Bioverfügbarkeit.

Besonders bevorzugt ist das erfindungsgemäße Verfahren bei der Herstellung von Zubereitungen mit kontrollierter Wirkstoffabgabe, insbesondere für Retardzubereitungen nützlich, die den Wirkstoff linear über mehrere Stunden freisetzen sollen.

### Beispiele 1 bis 4

Zur Herstellung der erfindungsgemäßen Zubereitungen wurden die folgenden, kommerziell erhältlichen Polymeren verwendet:

Polymer (III): Polyvinylpyrrolidon K 25 (Kollidon® 25)
Polyvinylpyrrolidon K 90 (Luviskol® K90)

Polymer (II): Hydroxypropylmethylcellulose Typ 2910 USP der Viskositätsstufe 50 mPa • s (Metolose® 60 SH 50)

Wirkstoff (I): Als Wirkstoff wurde Nimodipin verwendet.

Die in Tabelle 1 unter den Beispielen 1 bis 4 angegebenen Substanzen wurden gemischt und mittels einer gängigen Tablettenpresse Tabletten vom angegebenen Format und Gewicht hergestellt. Die thermische Aktivierung erfolgte 4 Stunden bei 140°C. Die Wirkstofffreisetzung wurde in einer gängigen Freisetzungsapparatur (Rührflügelmethode) bestimmt. Dazu wurden die Tabletten in Puffer pH = 6,8 bei 37°C und 150 Upm inkubiert. Innerhalb von 6 Stunden setzen die Tabletten den Wirkstoff gemäß Tabelle 1 frei.

Tabelle 1

| Beispiel | 1 | 2 | 3 | 4/100 Upm |
|---|---|---|---|---|
| Arzneistoff (I) | Nimodipin 90 mg (33,33 %) | Nimodipin 90 mg (33,33 %) | Nimodipin 90 mg (33,0 %) | Nimodipin 45 mg (33,3 %) |
| Polymer (III) | PVP K 90 66,22 mg (24,5 %) | PVP K 90 107,57 mg (39,8 %) | PVP K 90 86,46 mg (31,7 %) | PVP K 90 53,81 mg (39,8 %) |
| Polymer (II) | Metolose® 60 SH 50 113,24 mg (41,9 %) | Metolose® 60 SH 50 71,89 mg (26,6 %) | Metolose® 60 SH 50 95,46 mg (35 %) | Metolose® 60 SH 50 35,96 mg (26,6 %) |
| Mg-stearat | 0,81 mg (0,3 %) | 0,81 mg (0,3 %) | 0,82 mg (0,3 %) | 0,41 mg (0,3 %) |
| Tbl.-gewicht | 270,27 mg | 270,27 mg | 272,73 mg | 135,18 mg |
| Tbl.-format | 10 r 15 | 10 r 15 | 10 r 15 | 8 r 12 |
| Freisetzung | | | | |
| 60 min. | 16 % | 30 % | 22 % | 31 % |
| 120 min. | 34 % | 54 % | 44 % | 60 % |
| 180 min. | 52 % | 73 % | 63 % | 83 % |
| 240 min. | 66 % | 86 % | 77 % | 95 % |
| 300 min. | 78 % | 90 % | 87 % | 99 % |
| 360 min. | 87 % | 92 % | 93 % | 100 % |

Alle aufgeführten Beispielrezepturen zeigen eine nahezu vollständige gleichmäßige (lineare) Wirkstofffreisetzung über 6 Stunden. Die verwendeten Polyvinyllactame bewirken eine effektive Lösungsvermittlung und zusammen mit der eingesetzten Hydroxypropylcellulose eine Retardierung der Wirkstofffreisetzung über mehrere Stunden. Durch die Verwendung hochmolekularer Polyvinyllactame können auch hohe Arzneistoffdosen bei einem niedrigen Tablettengewicht effektiv lösungsvermittelt werden. Daher ist eine gute Schluckbarkeit der Tabletten gewährleistet.

**Vergleichbeispiel 1 zu Beispiel-Nr. 4** (ohne thermische Amorphisierung)

Die Rezeptur aus Beispiel 4 wurde unter identischen Bedingungen tablettiert, jedoch ohne anschließend die thermische Amorphisierung durchzuführen. Die Wirkstofffreisetzung erfolgte unter identischen Bedingungen wie im Vorangehenden beschrieben.

Tabelle 2

| Vergleichsbeispiel | 1 |
|---|---|
| Arzneistoff (I) | Nimodipin 45 mg (33,3 %) |
| Polymer (III) | PVP K 90 53,81 mg (39,8 %) |
| Polymer (II) | HPMC 60 SH 50 35,96 mg (26,6 %) |
| Mg-stearat | 0,41 mg (0,3 %) |
| Tbl.-gewicht | 135,18 mg |
| Tbl.-format | 8 r 12 |
| Freisetzung | |
| 60 min. | 5 % |
| 120 min. | 12 % |
| 180 min. | 18 % |
| 240 min. | 21 % |
| 300 min. | 25 % |
| 360 min. | 25 % |

Die Tablette setzt nur äußerst unvollständig den enthaltenen Wirkstoff frei.

**Vergleichsbeispiel 2 zu Beispiel Nr. 1 bis 4** (Lösungsmittelverfahren)

Es wurde eine wirkstoffhaltige Tablette durch ein Lösungsmittelverfahren hergestellt. Im einzelnen wurde eine acetonische Lösung von Nimodipin in PVP K 25 auf Crospovidone M aufgezogen und anschließend mit Metolose® 60 SH 50 und Milchzucker tablettiert. Es wurde keine thermische Amorphisierung durchgeführt. Die Wirkstofffreisetzung erfolgte unter identischen Bedingungen wie im Vorangehenden beschrieben.

Tabelle 3

| Vergleichsbeispiel | 2 |
|---|---|
| Arzneistoff (I) | Nimodipin 45 mg (11,75 %) |
| Polymer (III) | PVP K 25 112,5 mg (29,37 %) |
| | Crospovidone M 45 mg (11,75 %) |
| Polymer (II) | Metolose® 60 SH 50 160 mg (41,78 %) |
| | Milchzucker 19,5 mg (5,09 %) |
| Mg-stearat | 1 mg (0,26 %) |
| Tablettengewicht | 383 mg |
| Tablettenformat | 11 r 18 |
| Freisetzung | |
| 60 min. | 17 % |
| 120 min. | 43 % |
| 180 min. | 68 % |
| 240 min. | 90 % |
| 300 min. | 102 % |
| 360 min. | 101 % |

Die Tablette setzt den enthaltenen Wirkstoff vollständig frei. Das Herstellungsverfahren muß während der acetonischen Granulierung aufwendig unter Explosionsschutz in einer Vakuum-Wirbelschicht erfolgen. Die Tablette ist nahezu dreimal so schwer und deutlich größer als die nach dem erfindungsgemäßen Verfahren hergestellten Tabletten der Beispiele 1 bis 4.

**Vergleichsbeispiel 3 zu Beispiel Nr. 1 bis 4** (Extrusionsverfahren):

Es wurde eine wirkstoffhaltige Tablette durch ein Extrusionsverfahren hergestellt. Im einzelnen wurde eine Mischung aus Nimodipin, PVP K 25 und Crospovidone M durch eine Gegendrallzweischneckenmaschine bei 156°C extrudiert und anschließend mit Metolose® 60 SH 50 und Milchzucker tablettiert. Es wurde keine thermische Behandlung der Tabletten durchgeführt. Die Wirkstofffreisetzung erfolgte unter identischen Bedingungen wie im Vorangehenden beschrieben.

Tabelle 4

| Vergleichsbeispiel | 3 |
|---|---|
| Arzneistoff (I) | Nimodipin 45 mg (11,75 %) |
| Polymer (III) | PVP K 25 112,5 mg (29,37 %) |
| | Crospovidone M 45 mg (11,75 %) |
| Polymer (II) | Metolose® 60 SH 50 160 mg (41,78 %) |
| | Milchzucker 19,5 mg (5,09 %) |
| Mg-stearat | 1 mg (0,26 %) |
| Tablettengewicht | 383 mg |
| Tablettenformat | 11 r 18 |
| Freisetzung | |
| 60 min. | 26 % |
| 120 min. | 61 % |
| 180 min. | 88 % |
| 240 min. | 90 % |
| 300 min. | 91 % |
| 360 min. | 90 % |

Die Tablette setzt den enthaltenen Wirkstoff nahezu vollständig frei. Zur Erzielung des Freisetzungsprofiles ist im Vergleich zu Beispiel 4 etwa 3,8 mal mehr Polymermasse erforderlich. Das Herstellungsverfahren ist apparativ aufwendig.

**Beispiele 5 und 6**

Zur Herstellung der erfindungsgemäßen Zubereitungen wurden die folgenden, kommerziell erhältlichen Polymeren verwendet:

Polymer (III)      Polyvinylpyrrolidon K 90
(Luviskol® K90)
Copolymerisat aus 60 % N-Vinylpyrrolidon und 40 % Vinylacetat
(Kollidon® VA 64)

Polymer (II)      Hydroxypropylmethylcellulose
Typ 2910 USP der Viskositätsstufe 50 mPa·s (Metolose® 60 SH 50)
Hydroxypropylmethylcellulose Typ 2906 USP der Viskositätsstufe 4000 mPa·s (Metolose® 65 SH 4000)

Die in Tabelle 5 unter den Beispielen 5 und 6 angegebenen Substanzen wurden gemischt und mittels einer gängigen Tablettenpresse Tabletten vom angegebenen Format und Gewicht hergestellt. Die thermische Aktivierung erfolgte 2 Stunden bei 170°C. Die Wirkstofffreisetzung wurde in einer gängigen Freisetzungsapparatur durch die Rührflugelmethode bestimmt. Dazu wurden die Tabletten in Puffer pH 6,8 bei 37°C und 100 Upm inkubiert. Innerhalb von 8 Stunden setzten die Tabletten den Wirkstoff gemäß Tabelle 5 frei.

Tabelle 5

| Vergleichsbeispiel | 5 | 6 |
|---|---|---|
| Arzneistoff (I) | Nifedipin 62,5 mg (24,8 %) | Nifedipin 60,0 mg (20 %) |
| Polymer (III) | PVP K 90 94,125 mg (37,35 %) | Kollidon$^®$ VA 64 119,25 mg (39,75 %) |
| Polymer (II) | Metolose$^®$ 60 SH 50 94,125 mg (37,35 %) | Metolose$^®$ 65 SH 4000 119,25 mg (39,75 %) |
| Mg-stearat | 1,25 mg (0,5 %) | 1,5 mg (0,5 %) |
| Tablettengewicht | 252 mg | 300 mg |
| Tablettenformat | 10 mm | 10 mm |
| Freisetzung | | |
| 60 min. | 8 % | 6 % |
| 120 min. | 12 % | 11 % |
| 180 min. | 16 % | 17 % |
| 240 min. | 19 % | 25 % |
| 300 min. | 21 % | 35 % |
| 360 min. | 24 % | 46 % |
| 420 min. | 25 % | 57 % |
| 480 min. | 27 % | 67 % |

**Patentansprüche**

1. Verfahren zur Herstellung von festen Arzneizubereitungen mit kontrollierter Wirkstoffabgabe und hoher Stabilität, enthaltend ein Gewichtsteil eines oder mehrerer schwerlöslischer Wirkstoffe (I), 0,5 bis 50 Gew.-Teile eines Celluloseethers (II) und 0,5 bis 50 Gew.-Teile Polyvinyllactame oder Polyvinyllactam-Copolymere (III) sowie gegebenenfalls weitere Hilfsstoffe, dadurch gekennzeichnet, daß man die Bestandteile (I), (II) und (III) in Abwesenheit von Lösungsmitteln mischt und zu Arzneizubereitungen formt und diese anschließend über mindestens 30 Minuten bei Temperaturen zwischen 50 und 200°C thermisch aktiviert.

2. Verfahren gemäß Anspruch 1, dadurch gekennzeichnet, daß man die aus den drei Bestandteilen geformten Arzneizubereitungen durch Erhitzen auf 80 bis 170°C für einen Zeitraum von 30 Minuten bis 48 Stunden thermisch aktiviert.

3. Verfahren gemäß Anspruch 1, dadurch gekennzeichnet, daß man als Bestandteil (II) Celluloseether mit Methoxy- und Hydroxypropylsubstituenten einsetzt, die in 2 %iger wäßriger Lösung eine Viskosität von 15 bis 100 000 mPa.s besitzen.

4. Verfahren gemäß Anspruch 1, dadurch gekennzeichnet, daß man als Bestandteile (III) Polyvinylpyrrolidon oder Vinylpyrrolidon-Vinylacetat-Copolymerisat einsetzt, die ein mittleres Molekulargewicht von 20 000 bis 2 000 000 $\overline{M}$w besitzen.

5. Verfahren gemäß Anspruch 1, dadurch gekennzeichnet, daß als schwerlösliche Wirkstoffe Dihydropyridine eingesetzt werden.

6. Feste thermisch aktivierte Arzneizubereitungen erhältlich gemäß Anspruch 1.